# EUROPÄISCHE PATENTSCHRIFT

(11) **EP 1 383 752 B1**
(45) Veröffentlichungstag und Bekanntmachung des Hinweises auf die Patenterteilung: **22.06.2005**
(21) Anmeldenummer: 02727303.6
(22) Anmeldetag: 29.04.2002
(51) Int. Cl.: C07D 295/10, C07B 59/00, A61K 31/4453, A61P 9/08

(54) **DEUTERIERTE 3-PIPERIDINOPROPIOPHENONE SOWIE DIESE VERBINDUNGEN ENTHALTENDE ARZNEIMITTEL**
DEUTERATED 3-PIPERIDINOPROPIOPHENONE AND MEDICAMENTS CONTAINING SAID COMPOUNDS
3-PIPERIDINOPROPIOPHENONES DOPEES ET PRODUITS PHARMACEUTIQUES CONTENANT CES COMPOSES

(30) Priorität: 02.05.2001 DE 10123129
(43) Veröffentlichungstag der Anmeldung: 28.01.2004
(73) Patentinhaber: Turicum Drug Development AG, 6300 Zug (CH)
(72) Erfinder: ALKEN, Rudolf-Giesbert, 16366 Neuenhagen (DE); STABINGIS, Thomas, 10709 Berlin (DE)
(74) Vertreter: Schubert, Klemens, Dr.
(86) Internationale Anmeldenummer: PCT/DE2002/001607
(87) Internationale Veröffentlichungsnummer: WO 2002/088100

(56) Entgegenhaltungen:
- US-A- 4 638 009
- AXEL DIETRICH: "TOLPERISON: ALTER WIRKSTOFF ODER NEUE LEITSTRUKTUR" 1999 , DISSERTATION , PADERBORN XP002232677 cited in the application 3',5'-Dideutero-tolperison example 1A

## Beschreibung

Die Erfindung betrifft deuterierte 3-Piperidinopropiophenone und deren physiologisch verträgliche Salze sowie diese Verbindungen enthaltende Arzneimittel.

Bekannte Vertreter von 3-Piperidinopropiophenonen sind Tolperison und Eperison (US-A 3,995,047, US-A 4,638,009). Diese Verbindungen werden als Spasmolytika und Vasodilatatoren verwendet.

Aufgabe der vorliegenden Erfindung ist es, 3-Piperidinopropiophenone bereitzustellen, die gegenüber den bereits bekannten Verbindungen verbesserte pharmokinetische und/oder pharmakodynamische Eigenschaften aufweisen.

Überraschenderweise wurde nun gefunden, dass die erfindungsgemäßen deuterierten 3-Piperidinopropiophenone wesentlich bessere pharmakokinetische und/oder pharmakodynamische Eigenschaften aufweisen als die undeuterierten Verbindungen.

Erfindungsgemäß wird die Aufgabe also gelöst durch die Bereitstellung deuterierter 3-Piperidinopropiophenone der allgemeinen Formel I 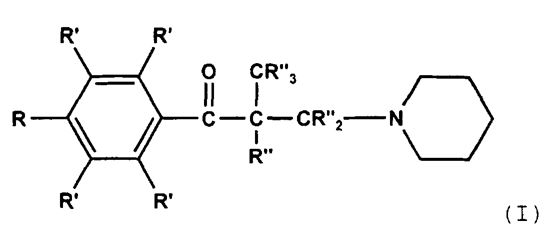 worin
R einen undeuterierten, einen ein- oder mehrfach deuterierten oder einen perdeuterierten Alkylrest mit bis zu 3 C-Atomen darstellt,
die Reste R' jeweils alle Wasserstoff sind oder gemeinsam alle Deuterium darstellen,
die Reste R'' unabhängig voneinander Deuterium oder Wasserstoff sind und wobei mindestens einer der Reste R, R' oder R'' Deuterium ist oder Deuterium enthält, sowie deren physiologisch verträgliche Salze.

Bevorzugt sind folgende erfindungsgemäße deuterierte 3-Piperidinopropiophenone:
4'-Deuteromethyl-2-methyl-3-piperidino-propiophenon,
4'-Methyl-2',3',5',6'-tetradeutero-2-methyl-3-piperidinopropiophenon,
4'-Ethyl-2',3',5',6'-tetradeutero-2-methyl-3-piperidinopropiophenon,
4'-iso-Propyl-2',3',5',6'-tetradeutero-2-methyl-3-piperidino-propiophenon,
4'-n-Propyl-2',3',5',6'-tetradeutero-2-methyl-3-piperidino-propiophenon,
4'-Trideuteromethyl-2',3',5',6'-tetradeutero-2-methyl-3-piperidino-propiophenon,
4'-Methyl-2-deuteromethyl-2-deuterium-3-piperidino-propiophenon
4'-Methyl-2-deuteromethyl-2-deuterium-3,3-dideutero-3-piperidino-propiophenon und
4'-Trideuteromethyl-2',3',5',6'-tetradeutero-2-methyl-3,3-dideutero-3-piperidino-propiophenon.

Bevorzugt ist die Verwendung der erfindungsgemäßen deuterierter 3-Piperidinopropiophenone sowie deren physiologisch verträglicher Salze, zur Behandlung von Erkrankungen mit Symptomen im muskulären Bereich.

Insbesondere bevorzugt ist die Verwendung deuterierter 3-Piperidinopropiophenone sowie deren physiologisch verträglicher Salze, zur Herstellung von Arzneimitteln zur Behandlung von Erkrankungen mit Symptomen im muskulären Bereich.

Besonders bevorzugt sind pharmazeutische Zusammensetzungen, welche die erfindungsgemäßen deuterierten 3-Piperdinopropiophenone sowie deren physiologisch verträgliche Salze zur Behandlung von Erkrankungen mit Symptomen im muskulären Bereich neben pharmazeutisch verträglichen Hilfs- und/oder Zusatzstoffen enthalten.

Die Herstellung der erfindungsgemäß verwendeten deuterierten Tolperisone ist an sich bekannt. Die als Ausgangsverbindung eingesetzten deuterierten Propiophenone wurden unter Verwendung von deuterierten Toluolderivaten durch Friedel-Crafts-Acylierung mit Propionsäurechlorid hergestellt (Organikum, 15. Auflage, 1977, S. 404-405). Verwendet wurden hierbei die kommerziell erhältlichen Toluolderivate Trideuteromethylbenzol, Perdeuterotoluol und das bekannte 2,3,4,5,6-Pentadeuterotoluol (A. Borovik et al., Angew. Chem., Int. Ed., 2000, 39(22), 4117-4118).

Die Umsetzung zu den deuterierten Tolperisonderivaten kann analog zu den bekannten Synthesen für 3H-Tolperison erfolgen (Dietrich, A.; Fels, G.; J. Labelled Compd. Radiopharm. (1999), 42(12), 1125-1134 sowie Dietrich, A. Dissertation 1999, Univ.-GH Paderborn).

A. Dierich beschreibt in diesen Arbeiten u. a. die Synthese von in 3'-Position und in 3',5'-Position tritiierten und deuterierten Tolperisonderivaten. Verwendet wurden diese Substanzen zur Untersuchung der Wirkungsweise und Pharmakologie von Tolperison.

Die in der 2-Position und 2-Methylposition deuterierten Tolperisonderivate wurden ausgehend vom bekannten 2,3-Didehydro-tolperison (Dietrich, A. Dissertation 1999, U-niv.-GH Paderborn) durch Umsetzung mit Deuterium erzeugt. Die in der 1-Position deuterierten Verbindungen wurden auf an sich bekanntem Weg durch Verwendung von deuteriertem Paraformaldehyd bei der Mannichreaktion mit den entsprechenden Propiophenonderivaten erhalten.

Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beispielweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wässrige Lösungen von Säureadditionssalzen der erfindungsgemäß verwendeten Verbindungen in einer wässrigen Säurelösung hergestellt werden.

Die Säureadditionssalze der erfindungsgemäßen Verbindungen können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, topischen (cutan, transdermal, lokal), subcutanen, intravenösen oder intramuskulären Applikation, die neben üblichen Träger- und Verdünnungsmitteln eine Verbindung der allgemeinen Formel I oder deren Säureadditionssalz als Wirkstoff enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Die topische Anwendung kann beispielsweise in der Form von Salben, Cremes, Gelen, Lösungen oder durch Pflaster erfolgen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden.

Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß verwendeten Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die Herstellung der erfindungsgemäßen Arzneimittel zur topischen Applikation ist dem Fachmann bekannt. Bei der Herstellung der erfindungsgemäßen Arzneimittel zur transdermalen Anwendung werden die an sich bekannten Hilfs- und Enhancerstoffe verwendet.

Die Herstellung der erfindungsgemäßen pharmazeutischen Zubereitungen ist an sich bekannt und in den dem Fachmann bekannten Handbüchern beschrieben, beispielsweise Hager's Handbuch (5.) 2, 622-1045; List et al., Arzneiformenlehre, Stuttgart: Wiss. Verlagsges. 1985; Sucker et al., Pharmazeutische Technologie, Stuttgart: Thieme 1991; Ullmann's Enzyklopädie (5.) A 19, 241-271; Voigt, Pharmazeutische Technologie, Berlin: Ullstein Mosby 1995.

Die so hergestellten Arzneimittel können zur Behandlung von Erkrankungen mit Symptomen im muskulären Bereich verwendet werden.

Die erfindungsgemäßen Verbindungen weisen gegenüber den im Stand der Technik bekannten Verbindungen, welche kein Deuterium tragen eine Reihe von Vorteilen auf. Durch die Deuterierung wird zum einen die Metabolisierung im Organismus verändert. Insbesondere wird die Hydroxylierung am Phenylrest erschwert, was zu einem verminderten First-Pass Effekt führt. Hierdurch ist es möglich, die Dosierung zu verändern und länger wirkende Zubereitungen zu schaffen, welche auch in Form von Depotzubereitungen die Compliance verbessern können.

Daneben ist auch die Pharmakodynamik verändert, weil die deuterierten Verbindungen völlig andere Hydrathüllen ausbilden, so dass die Verteilung im Organismus sich von der der undeuterierten Verbindungen deutlich unterscheidet.

Die Metabolisierung von Tolperison und davon abgeleiteten Substanzen erfolgt vorwiegend in der Leber, wobei ein starker First-Pass Effekt zu beobachten ist. Lediglich ein Fünftel der zugeführten Dosis findet sich unverändert im Blut wieder.

Maßgeblich für die hepatische Metabolisierung von Medikamenten bzw. Xenobiotika sind Cytochrom-P450 (CYP) Enzyme. Die primären Metaboliten beim hepatischen Abbau entstehen durch Hydroxylierung des am aromatischen Ring befindlichen Alkylsubstituenten und durch Hydroxylierung des Aromaten selbst (Miyazaki, Ishibashi, Takayama; 4th Symposium on Drug Metabolism and Action, 1972, Sendai; Japan: 154 - 164).

Um nähere Erkenntnisse zur hepatischen Metabolisierung von Tolperison und der beanspruchten deuterierten Analoga zu erhalten, wurden pharmakokinetische in-vitro Studien mit den dem Durchschnittsfachmann bekannten, in der Leber am häufigsten anzutreffenden Cytochrom P450 Familien (CYP1A1, CYP1A2, CYP2C8, CYP2C19, CYP2D6, CYP2E1, CYP3A4) durchgeführt.

Die Ergebnisse dieser Untersuchungen sind in den Figuren 1 bis 3 dargestellt.

Es zeigt:
Fig. 1 den enzymatischen Abbau von Tolperison durch Cytochrom-Oxidasen;
Fig. 2 die Bildung von Hydroxy-Metaboliten aus Tolperison durch CYP2D6 und CYP2C19 im Vergleich mit weniger aktiven Cytochromen und
Fig. 3 die Bildung von Hydroxy-Metaboliten aus Verbindung **I** durch Cytochrom-P450 Enzyme.

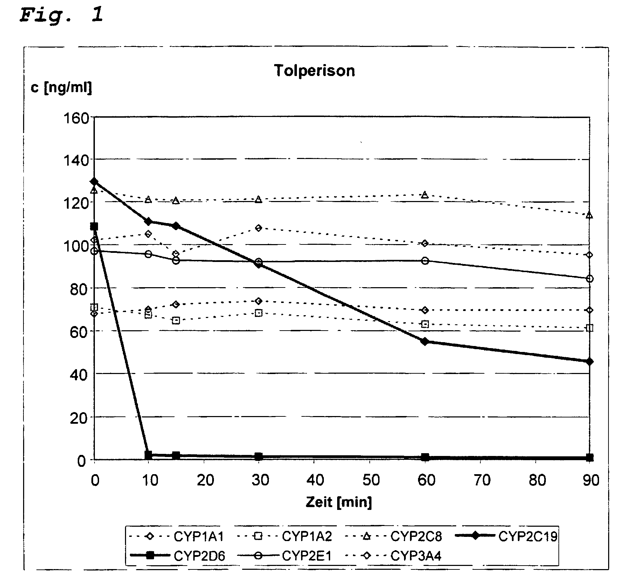

Figur 1 zeigt die unterschiedlichen Abnahmen der Tolperisonkonzentration durch enzymatischen Abbau mittels Cytochrom-Oxidasen über die Zeit.

Demnach wird Tolperison vor allem durch CYP2C19 und CYP2D6 umgewandelt. Die anderen untersuchten Cytochrome tragen allenfalls unwesentlich zum biologischen Abbau von Tolperison bei.

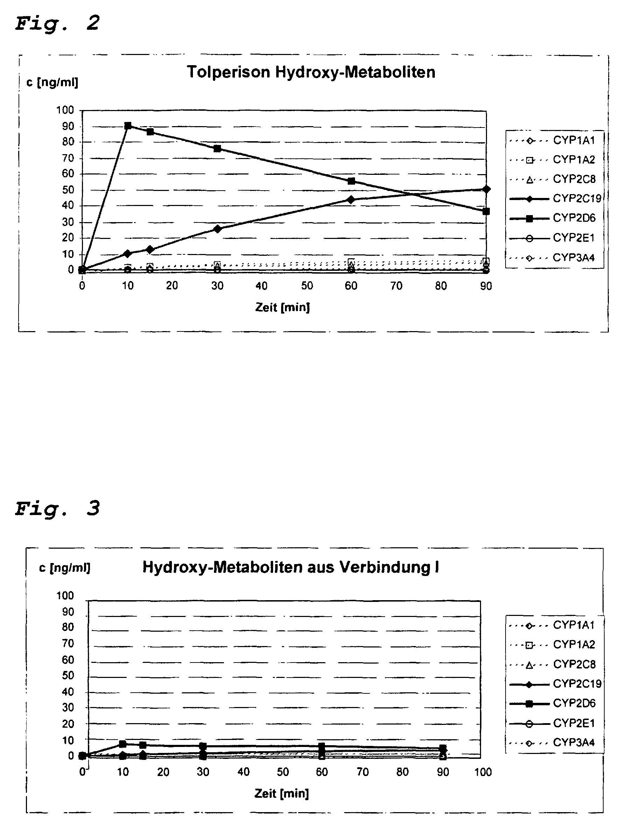

Die durch Hydroxylierung entstehenden primären Metaboliten werden durch CYP2C19 und CYP2D6 im selben Maße gebildet, wie das Substrat abgebaut wird (siehe Figur 2).

Überraschenderweise unterscheidet sich die hepatische Metabolisierung der erfindungsgemäßen deuterierten Verbindungen durch Cytochrom-P450-Oxidasen deutlich von der der entsprechenden undeuterierten Substanzen.

So wird beispielsweise die enzymatische Hydroxylierung von 1-[4-(Trideuteromethyl)tetradeuterophenyl]-2-methyl-3-piperidin-1-yl-1-propanon (**I**; Formel I mit R = CD₃, R' = D, R'' = H) durch CYP2C19 und CYP2D6 im Vergleich zu Tolperison effektiv um den Faktor 10 verzögert (siehe Figur 3).

Hieraus ergibt sich eine Verbesserung der Wirksamkeit, da von einer Verlängerung der Wirkdauer ausgegangen werden muss. Der therapeutische Nutzen liegt in einer Dosisreduzierung bei der Verwendung von aus den erfindungsgemäßen deuterierten Propiophenonen hergestellten Arzneimitteln gegenüber den bislang verwendeten nichtdeuterierten analogen Verbindungen.

Damit ist es möglich, völlig neuartige Zubereitungsformen zu entwickeln.

Die folgenden Beispiele erläutern die Erfindung:

### Beispiel 1

### Herstellung von 4'-Trideuteromethylpropiophenon

In einem Dreihalskolben mit Rührer, Tropftrichter und Rückflußkühler mit Calciumchloridtrockenrohr wurden 40 ml Dichlorethan mit 16 g wasserfreiem fein gepulvertem Aluminiumchlorid versetzt. Unter Eiskühlung wurden 13,88 g Propionsäurechlorid hinzugetropft. 9,5 g Trideuteromethylbenzol (Toluol-d3) wurden nun in einer Geschwindigkeit zugetropft, dass die Temperatur der Reaktionslösung konstant bei 20 °C gehalten wurde. Nach beendeter Zugabe wurde das Gemisch 2 Stunden lang gerührt und anschließend über Nacht stehen gelassen. Der entstandene Keton-Aluminiumchlorid-Komplex wurde zerstört, indem der Reaktionsansatz vorsichtig auf 50 ml Eis gegossen wurde. Die organische Phase wurde abgetrennt und die wässrige Phase drei Mal mit Dichlorethan extrahiert. Die vereinigten organischen Extrakte wurden mit Wasser, mit 2%-iger Natronlauge und anschließend wieder mit Wasser gewaschen und dann über Kaliumcarbonat getrocknet.
Das Lösemittel wurde entfernt und der Rückstand im Vakuum destilliert.
Ausbeute: 10,2 g (68%) 4'-Trideuteromethylpropiophenon als farblose Flüssigkeit.

| C₁₀H₉D₃O: 151, 223 | | |
|---|---|---|
| ber. | C 79,43 | H 9,99 |
| gef. | C 79,41 | H 10,01 |

¹H-NMR: Beim Vergleich mit dem ¹H-NMR-Spektrum des nicht deuterierten 4'-Methylpropiophenons konnte beim ¹H-NMR-Spektrum des Produkts, bei sonstiger Übereinstimmung, die Abwesenheit des Resonanzsignals der Aromaten-CH₃-Gruppe festgestellt werden.

### Beispiel 2

### Herstellung von 4'-Trideuteromethyl-2',3',5',6'-tetradeuteropropiophenon

Analog zu Beispiel 1 wurden 16 g wasserfreies fein gepulvertes Aluminiumchlorid in 40 ml Dichlorethan unter Eiskühlung mit 13,88 g Propionsäurechlorid versetzt und mit 10,02 g Trideuteromethyl-2,3,4,5,6-tetradeuterobenzol (Toluol-d8) zur Reaktion gebracht.
Abweichend wurde hier jedoch der Keton-Aluminiumchlorid-Komplex durch Eingießen in eisgekühltes D₂O zerstört. Die weitere Aufarbeitung erfolgte analog zu Beispiel 1. Ausbeute: 10,24 g (66%) 4'-Trideuteromethyl-2',3',5',6'-tetradeuterophenylpropiophenon als farblose Flüssigkeit.

| C₁₀H₅D₇O: | | |
|---|---|---|
| ber. | C 77,37 | H 12,33 |
| gef. | C 77,40 | H 12,31 |

¹H-NMR: Beim Vergleich mit dem ¹H-NMR-Spektrum des nicht deuterierten 4'-Methylpropiophenons konnte beim ¹H-NMR-Spektrum des Produkts, bei sonstiger Übereinstimmung, die Abwesenheit des Resonanzsignals der Aromaten-CH₃-Gruppe sowie der Aromatenprotonen festgestellt werden.

### Beispiel 3

### Herstellung von 4'-Methyl-2',3',5',6'-tetradeuteropropiophenon

Analog zu Beispiel 2 wurden 16 g wasserfreies fein gepulvertes Aluminiumchlorid in 40 ml Dichlorethan unter Eiskühlung mit 13,88 g Propionsäurechlorid versetzt und mit 9,72 g 2,3,4,5,6-Pentadeuterotoluol (Toluol-d5) zur Reaktion gebracht. Die Aufarbeitung erfolgte wie in Beispiel 1 beschrieben.
Ausbeute: 9,59 g (63%) 4'-Methyl-2',3',5',6'-tetradeuteropropiophenon als farblose Flüssigkeit.

| C₁₀H₈D₄O | | |
|---|---|---|
| ber. | C 78,9 | H 10,59 |
| gef. | C 79,3 | H 10,53 |

¹H-NMR: Beim Vergleich mit dem ¹H-NMR-Spektrum der nicht deuterierten 4'-Methylpropiophenon konnte beim ¹H-NMR-Spektrum des Produkts, bei sonstiger Übereinstimmung, die Abwesenheit des Resonanzsignals der Aromatenprotonen festgestellt werden.

### Beispiel 4

### Herstellung von 4'-Trideuteromethyl-2-methyl-3-piperidino-propiophenon

1,15 g 4'-Trideuteromethylpropiophenon wurden in 5 ml Methanol gelöst und anschließend 0,3 g Paraformaldehyd und 1,1 g Piperidinhydrochlorid unter Rühren zugefügt. Das Reaktionsgemisch wurde auf Rückfluß erhitzt, bis der Reaktionsendpunkt erreicht war (Erhärtung des Reaktionsgemisches). Anschließend wurden 10 ml Chloroform hinzugefügt, die organischen Phasen über Natriumsulfat getrocknet, filtriert und das Lösungsmittel im Vakuum abgezogen. Der erhaltene Feststoff wurde fein zerkleinert und mit Aceton gewaschen. Die erhaltenen 1,5 g des kristallinen Rohproduktes wurden in das Hydrochlorid überführt, welches aus Methanol umkristallisiert wurde. Ausbeute: 1,45 g (73%) in Form von Nadeln.
Schmp: 167-169°C.

¹H-NMR (200 MHz, CDCl₃): δ = 1,18 (d, 3H, CH₃), 1,25-1,68 (m, 6H, 3 x CH₂), 2,18-2,45 (m, 4H, 2 x CH₂), 2,35 und 2,65 (d und AB-Spektrum, *J* = 7,1 Hz, *J*_{A,B} = 12,4 Hz, 2H, CH₂), 3,69 (m, 1H, CH), 7,78 (s, 4H, Ar-H).

¹³C-NMR (50 MHz, CDCl₃): δ = 18,00 (Ar-CD₃), 20,01 (CH₃), 22,15 (CH₂), 23,15 (CH₂), 34,25 (CH), 52,67 (2 CH₂), 58,53 (CH₂), 126,73 (Cₐᵣₒₘ), 129,42 (Cₐᵣₒₘ), 131,34 (Cₐᵣₒₘ), 155,02 (Cₐᵣₒₘ), 204,02 (C:O).

| C₁₆H₂₀NOD₃.HCl (284,85): | | | |
|---|---|---|---|
| ber. | C 67,47 | H 9,55 | N 4,92 |
| gef. | C 67,45 | H 9,56 | N 4,91 |

### Beispiel 5

### Herstellung von 4'-Methyl-2',3',5',6'-tetradeutero-2-methyl-3-piperidino-propiophenon

Analog zu Beispiel 4 wurden 1,16 g 4'-Methyl-2',3',5'6'-tetradeuteropropiophenon in 5 ml Methanol gelöst und anschließend mit 0,3 g Paraformaldehyd und 1,1 g Piperidinhydrochlorid zur Reaktion gebracht. Das Produkt wurde als Hydrochlorid isoliert.
Ausbeute: 1,42 g (71%) in Form von Nadeln.
Schmp: 174-176°C

¹H-NMR (200 MHz, CDCl₃): δ = 1,18 (d, 3H, CH₃), 1,25-1,68 (m, 6H, 3 CH₂), 2,18-2,45 (m, 4H, 2 CH₂), 2,35 und 2,65 (d und AB-Spektrum, *J* = 7,1 Hz, *J*_{A,B} = 12,4 Hz, 2H, CH₂), 2,41 (s, 3H, Ar-CH₃), 3,69 (m, 1H, CH).

| C₁₆H₁₉NOD₄.HCl (285,85) | | | |
|---|---|---|---|
| ber. | C 67,23 | H 9,87 | N 4,9 |
| gef. | C 67,21 | H 9,89 | N 4,8 |

### Beispiel 6

### Herstellung von 4'-Trideuteromethyl-2',3',5',6'-tetradeutero-2-methyl-3-piperidino-propiophenon

Analog Beispiel 4 wurden 1,09 g 4'-Trideuteromethyl-2',3',5'6'-tetradeuteropropiophenon in 5 ml Methanol gelöst und anschließend mit 0,3 g Paraformaldehyd und 1,1 g Piperidinhydrochlorid zur Reaktion gebracht. Das Produkt wurde als Hydrochlorid isoliert.
Ausbeute: 1,46 g (72 %) in Form von Nadeln
Schmp: 177-178°C

| C₁₆H₁₆NOD₇.HCl (288,87) | | | |
|---|---|---|---|
| ber. | C 66,53 | H 10,81 | N 4,85 |
| gef. | C 66,55 | H 10,84 | N 4,87 |

¹H-NMR (200 MHz, CDCl₃): δ = 1,18 (d, 3H, CH₃), 1,25-1,68 (m, 6H, 3 CH₂), 2,18-2,45 (m, 4H, 2 CH₂), 2,35 und 2,65 (d und AB-Spektrum, *J* = 7,1 Hz, *J*_{A,B} = 12,4 Hz, 2H, CH₂), 3,69 (m, 1H, CH).

IR: νₘₐₓ (Nujol) 2721, 2639, 2532, 2408, 1674 (C:O), 1580 (Ar), 1544, 1460, 1411, 1378, 1331, 1298, 1244, 1211, 1159, 1121, 1083, 1081, 1021, 721, 638 cm⁻¹.

### Beispiel 7

### Herstellung von 4'-Methyl-2-deuteromethyl-2-deuterium-3-piperidino-propiophenon

Zu einer Lösung von 10 g (41 mmol) 2,3-Didehydrotolperison in 150 ml Ethylacetat wurden 100 mg Pd/C (10 %) zugefügt und das Reaktionsgefäß mit Deuteriumgas gespült, welches dann an eine Parr-Apparatur angeschlossen wurde. Die Deuterierung erfolgt bei 2 atm bei Raumtemperatur über Nacht. Das Reaktionsgemisch wurde über Celite abfiltriert und das Filtrat im Vakuum eingeengt. Anschließend wurde der Rückstand mit 1N NaOH aufgenommen und mit Diethylether extrahiert, die organische Phase abgetrennt, über Natriumsulfat getrocknet, filtriert und im Vakuum eingeengt. Das resultierende Amin wurde in Diethylether gelöst und Acetylchlorid und Methanol hinzugefügt, um das Hydrochlorid herzustellen.
Ausbeute: 8,6 g (85%) des deuterierten Tolperisons wurden gewonnen.
Schmelzpunkt: 178°C.

¹H-NMR (200 MHz, CDCl₃): δ = 1.15 (d, 2H, CDH₂), 1.30 - 1.72 (m, 6H, 3 CH₂), 2.20 -2.48 (m, 4H, 2 CH₂), 2.48 (s, 3H, CH₃), 2.49 und 2.85 (d und AB-Spektrum, *J* = 7.2 Hz, *J*_{A,B} = 12,6 Hz, 2H, CH₂), 7.95 (s, 2H, Ar-H).

¹³C-NMR (50 MHz, CDCl₃): δ = 19.21 (Ar-CH₃), 18.78 (CDH₂), 22.20 (CH₂), 22.95 (CH₂), 35.32 (CD), 52.53 (2 CH₂), 58.60 (CH₂), 129.33 (Cₐᵣₒₘ), 130.32 (Cₐᵣₒₘ), 132.15 (Cₐᵣₒₘ), 145.55 (S, Cₐᵣₒₘ), 201.02 (C:O).

| C₁₆H₂₁NOD₂.HCl (283.48) : | | | |
|---|---|---|---|
| ber. | C 67,71 | H 9,23 | N 4,93 |
| gef. | C 67,73 | H 9,21 | N 4,95 |

### Beispiel 8

### In vitro Versuche zum biologischen Abbau der Testsubstanzen durch Cytochrom P450 Enzyme

Verwendete Zelllinien: CYP1A1, CYP1A2, CYP2C8, CYP2C19, CYP2D6, CYP2E1, CYP3A4.
Inkubation bei 37°C in 200 µl Inkubationslösung bestehend aus 0,1 M Kaliumphosphat oder 0,5 M Tris.HCl Puffer (pH 7,4), 3 mM NADPH mit einer Proteinkonzentration von 0,5 mg/ml.
Die Analyse der Enzymtests erfolgte mittels LC/MS/MS.

## Patentansprüche

1. Deuterierte 3-Piperidinopropiophenone der allgemeinen Formel I 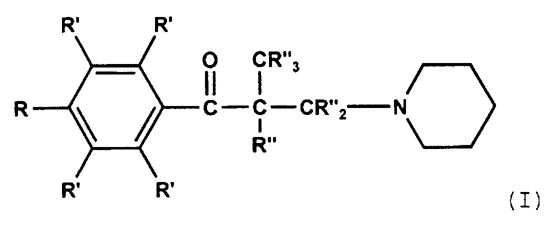 worin
R einen undeuterierten, einen ein- oder mehrfach deuterierten oder einen perdeuterierten Alkylrest mit bis zu 3 C-Atomen darstellt,
die Reste R' jeweils alle Wasserstoff sind oder jeweils alle Deuterium darstellen und
die Reste R" unabhängig voneinander Deuterium oder Wasserstoff sind
und wobei mindestens einer der Reste R, R' oder R" Deuterium ist oder Deuterium enthält,
sowie deren physiologisch verträgliche Salze.

2. Deuterierte 3-Piperidinopropiophenone gemäß Anspruch 1, nämlich
4'-Trideuteromethyl-2-methyl-3-piperidino-propiophenon,
4'-Methyl-2',3',5',6'-tetradeutero-2-methyl-3-piperidino-propiophenon,
4'-Ethyl-2',3',5',6'-tetradeutero-2-methyl-3-piperidino-propiophenon,
4'-iso-Propyl-2',3',5',6'-tetradeutero-2-methyl-3-piperidino-propiophenon,
4'-n-Propyl-2',3',5',6'-tetradeutero-2-methyl-3-piperidino-propiophenon,
4'-Trideuteromethyl-2',3',5',6'-tetradeutero-2-methyl-3-piperidino-propiophenon,
4'-Methyl-2-deuteromethyl-2-deuterium-3-piperidino-propiophenon
4'-Methyl-2-deuteromethyl-2-deuterium-3,3-dideutero-3-piperidino-propiophenon und
4'-Trideuteromethyl-2',3',5',6'-tetradeutero-2-methyl-3,3-dideutero-3-piperidino-propiophenon.

3. Verwendung der deuterierten 3-Piperidinopropiophenone gemäß Anspruch 1 oder 2 sowie deren physiologisch verträglicher Salze, zur Herstellung von Arzneimitteln zur Behandlung von Erkrankungen mit Symptomen im muskulären Bereich.

4. Pharmazeutische Zusammensetzung enthaltend deuterierte 3-Piperdinopropiophenone gemäß Anspruch 1 oder 2 sowie deren physiologisch verträgliche Salze zur Behandlung von Erkrankungen mit Symptomen im muskulären Bereich neben pharmazeutisch verträglichen Hilfs- und/oder Zusatzstoffen.

## Claims

1. Deuterated 3-piperidinopropiophenones of the general formula I, 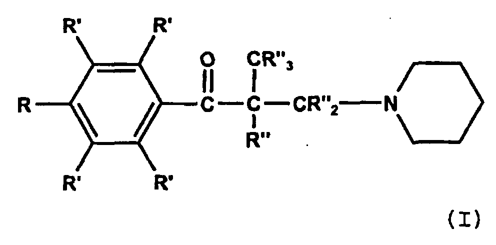 wherein
R represents an undeuterated, a mono- or polydeuterated, or a perdeuterated alkyl group containing up to 3 C atoms,
the groups R' are all hydrogen or all represent deuterium,
the groups R" are, independently of one another, deuterium or hydrogen,
and wherein at least one of the groups R, R', or R" is deuterium or contains deuterium,
as well as their physiologically tolerated salts.

2. The deuterated 3-piperidinopropiophenones according to claim 1, namely,
4'-trideuteromethyl-2-methyl-3-piperidinopropiophenone,
4'-methyl-2', 3', 5', 6'-tetradeutero-2-methyl-3-piperidinopropiophenone,
4'-ethyl-2', 3', 5', 6'-tetradeutero-2-methyl-3-piperidinopropiophenone,
4'-isopropyl-2', 3', 5', 6'-tetradeutero-2-methyl-3-piperidinopropiophenone,
4'-n-propyl-2', 3', 5', 6'-tetradeutero-2-methyl-3-piperidinopropiophenone,
4'-trideuteromethyl-2', 3', 5', 6'-tetradeutero-2-methyl-3-piperidinopropiophenone,
4'-methyl-2-deuteromethyl-2-deuterium-3-piperidinopropiophenone,
4'-methyl-2-deuteromethyl-2-deuterium-3,3-dideutero-3-piperidinopropiophenone, and
4'-trideuteromethyl-2', 3', 5', 6'-tetradeutero-2-methyl-3,3-dideutero-3-piperidinopropiophenone.

3. Use of the deuterated 3-piperidinopropiophenones according to claim 1 or 2 as well as their physiologically tolerated salts for the preparation of pharmaceutical drugs for the treatment of illnesses with symptoms in the muscular region.

4. Pharmaceutical formulation containing deuterated 3-piperidinopropiophenones according to claim 1 or 2 as well as their physiologically tolerated salts for the treatment of illnesses with symptoms in the muscular region in addition to containing pharmaceutically tolerated adjuvants and/or additives.

## Revendications

1. 3-pipéridinopropiophénones deutérées de formule générale I 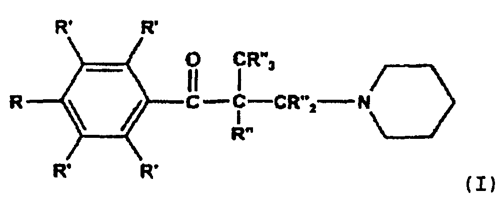 dans laquelle
R représente un radical alkyle non deutéré, une ou plusieurs fois deutéré ou perdeutéré avec jusqu'à 3 atomes de carbone,
les radicaux R' sont respectivement tous un hydrogène ou représentent respectivement tous un deutérium et
les radicaux R" sont indépendamment les uns des autres un deutérium ou un hydrogène
et dans laquelle au moins l'un des radicaux R, R' ou R" est un deutérium ou contient un deutérium,
ainsi que leurs sels physiologiquement acceptables.

2. 3-pipéridinopropiophénones deutérées selon la revendication 1, qui sont les
4'-trideutérométhyl-2-méthyl-3-pipéridino-propiophénone,
4'-méthyl-2',3',5',6'-tétradeutéro-2-méthyl-3-pipéridino-propiophénone,
4'-éthyl-2',3',5',6'-tétradeutéro-2-méthyl-3-pipéridino-propiophénone,
4'-isopropyl-2',3',5',6'-tétradeutéro-2-méthyl-3-pipéridino-propiophénone,
4'-n-propyl-2',3',5',6'-tétradeutéro- 2-méthyl-3-pipéridino-propiophénone,
4'-trideutérométhyl-2',3',5',6'-tétradeutéro-2-méthyl-3-pipéridino-propiophénone,
4'-méthyl-2-deutérométhyl-2-deutérium-3-pipéridino-propiophénone,
4'-méthyl-2-deutérométhyl-2-deutérium-3,3-dideutéro-3-pipéridino-propiophénone et
4'-trideutérométhyl-2',3',5',6'-tétradeutéro-2-méthyl-3,3-dideutéro-3-pipéridino-propiophénone.

3. Utilisation des 3-pipéridinopropiophénones deutérées selon la revendication 1 ou 2, ainsi que de ses sels physiologiquement acceptables, pour la préparation de médicaments pour le traitement de maladies avec des symptômes dans le domaine musculaire.

4. Composition pharmaceutique contenant des 3-pipéridinopropiophénones deutérées selon la revendication 1 ou 2, ainsi que ses sels physiologiquement acceptables, pour le traitement de maladies avec des symptômes dans le domaine musculaire en plus d'adjuvants et/ou additifs pharmaceutiquement acceptables.
